# EUROPÄISCHE PATENTSCHRIFT

(11) **EP 4 335 914 B1**
(45) Veröffentlichungstag und Bekanntmachung des Hinweises auf die Patenterteilung: **01.04.2026**
(21) Anmeldenummer: 22194253.5
(22) Anmeldetag: 07.09.2022
(51) Int. Cl.: C11D 3/386, C11D 11/00

(54) **TEXTILWASCHVERFAHREN**
TEXTILE WASHING METHOD
PROCÉDÉ DE LAVAGE DE TEXTILES

(43) Veröffentlichungstag der Anmeldung: 13.03.2024
(73) Patentinhaber: Henkel AG & Co. KGaA, 40589 Düsseldorf (DE)
(72) Erfinder: Kessler, Arnd, 40789 Monheim am Rhein (DE); Hardacker, Ingo, 46499 Hamminkeln (DE); Degering, Christian, 40699 Erkrath (DE); Wieland, Susanne, 41541 Zons/Dormagen (DE); Ruiz Hernandez, Robert, 40211 Düsseldorf (DE); Linz, Sergio, 50825 Köln (DE)

(56) Entgegenhaltungen:
- EP-B1- 2 566 943
- WO-A1-2020/208052
- WO-A1-2021/105919
- WO-A2-2021/122120
- US-A1- 2019 367 847
- NN: "Lavergy Pro 104 L", TECHNICAL INFORMATION, 1 May 2018 (2018-05-01), pages 1 - 3, XP093023716, Retrieved from the Internet <URL:https://assets.website-files.com/5fc9222730707183b6adc32a/60ad773f1856acb62e769cac_Lavergy-Pro-104-L-30663168.pdf> [retrieved on 20230214]

## Beschreibung

Die vorliegende Erfindung betrifft ein Textilwaschverfahren. Insbesondere betrifft die Anmeldung ein mehrstufiges maschinelles Textilwaschverfahren in dessen Verlauf wasch- und reinigungsaktive Substanzen zeitversetzt dosiert werden.

Während derzeit insgesamt weniger als ein Drittel der Menschheit Zugang zu einer Textilwaschmaschine haben, ist die maschinelle Textilreinigung seit den 1970er Jahren in einigen Regionen der Erde das Standardverfahren zur Entfernung von Verschmutzungen und zur Auffrischung der Wäsche.

Sowohl die Maschinentechnik als auch die in den maschinellen Textilreinigungsverfahren eingesetzten Waschmittel sind in den vergangenen Jahrzehnten kontinuierlich entwickelt und im Hinblick auf ihr Leistungsvermögen und ihren ökologischen Fußabdruck verbessert worden. Wenn die Entwicklungsbemühungen anfänglich auf die Verbesserung der Einzelbestandteile des Waschprozesses, beispielsweise die Textilwaschmaschine und deren Mechanik und Programme oder das Textilwaschmittel, gerichtet waren, steht in jüngster Zeit insbesondere auch die Verbesserung der Wechselwirkung dieser Bestandteile im Mittelpunkt des Interesses.

In der internationalen Anmeldung WO 2012/048911 A1 wird ein Waschverfahren unter Einsatz einer Textilwaschmaschine beschrieben, in dessen Verlauf die Textilien in einem Spülgang mit einer Spüllösung besprüht werden.

In dem europäischen Patent EP 3 428 336 B1 werden Waschverfahren mit einer Mindestdauer von 110

Minuten offenbart, in deren Rahmen der Waschflüssigkeit Verjüngungsmittel-haltige Waschmittel zugesetzt werden.

Das europäische Patent EP 2 711 413 B1 hat Waschverfahren zum Gegenstand, welche durch eine zeitversetzte Dosierung unterschiedlicher waschaktiver Substanzen gekennzeichnet sind.

Das europäische Patent EP 2 566 943 B1 offenbart die sensorgesteuerte zeitversetzte Dosierung von waschaktiven Substanzen, beispielsweise Peroxocarbonsäuren, in das Innere einer Textilwaschmaschine.

In der internationalen Anmeldung WO 2020/208052 A1 wird ein Waschverfahren beschrieben, bei dem Hilfsstoffe wie Enzyme zu einem anderen Zeitpunkt zugegeben werden als die Tenside.

Auch vor dem Hintergrund der bisherigen Entwicklungen besteht weiterhin die technische Aufgabe, die Waschleistung von Textilwaschmitteln in Textilwaschmaschinen zu verbessern.

Zur Lösung dieser Aufgabe ist ein Waschverfahren wie in Anspruch 1 definiert für Textilien in einer Trommelwaschmaschine, umfassend die Schritte
a) Bereitstellen einer Waschmaschine mit einem Waschprogramm, umfassend einen Hauptwaschgang der Dauer t_{w};
b) Einbringen von Textilien in den Wäschebehandlungsraum der Waschmaschine;
c) Einbringen einer wässrigen Flotte in den Wäschebehandlungsraum der Waschmaschine;
d) Einbringen einer ersten Waschmittelzusammensetzung wie in Anspruch 1 definiert in den Wäschebehandlungsraum der Waschmaschine im Hauptwaschgang zu einem Zeitpunkt 0 bis 10% t_{w};
e) Einbringen einer zweiten Waschmittelzusammensetzung, umfassend, bezogen auf ihr Gesamtgewicht,
   i) mindestens 4 Gew.-% aktives Protease-Protein
   ii) mindestens 80 Gew.-% eines flüssigen Trägers
   in die wässrige Flotte des Hauptwaschgangs zu einem Zeitpunkt 11 bis 99% t_{w}.

Als Waschmaschine wird ein motorbetriebenes Gerät zur Reinigung von Textilien bezeichnet. Bevorzugt sind insbesondere Trommelwaschmaschinen mit einer um eine horizontale Achse drehbaren Wäschetrommel. Das erfindungsgemäße Verfahren eignet sich insbesondere zur Durchführung in einer Trommelwaschmaschine mit einem Laugenbehälter, einer innerhalb des Laugenbehälters angebrachte Wäschetrommel, als Wäschebehandlungsraum, sowie einer Abpumpvorrichtung, die zum Abpumpen wässriger Flotte aus dem Laugenbehälter eingerichtet ist.

In Schritt a) des Verfahrens wird eine Waschmaschine mit einem Waschprogramm, umfassend einen Hauptwaschgang der Dauer t_{w} bereitgestellt. Übliche Waschmaschine verfügen in der Regel über eine Mehrzahl, für die Reinigung unterschiedlicher Textilien vorgesehener Waschprogramme, welche neben einem Hauptwaschgang über Vorspül-, Spül- und/oder Schleudergänge verfügen können. Bevorzugte Waschprogramme umfassen einen Hauptwaschgang, mindestens einen Spülgang und mindestens einen Schleudergang. Alternative Waschprogramme weisen mindestens einen Vorwaschgang, einen Hauptwaschgang, mindestens einen Spülgang und mindestens einen Schleudergang auf.

Neben der auf die Wäsche wirkenden mechanischen Kräfte, dem eingesetzten Waschmittel und der in der Waschflotte erreichten Flottentemperatur hat die Dauer des Waschgangs, insbesondere des Hauptwaschgangs Einfluss auf die erzielte Reinigungsleistung. Vorzugsweise beträgt die Dauer t_{w} des in dem Waschverfahren eingesetzten Hauptwaschgangs 15 bis 400 Minuten, vorzugsweise 30 bis 240 Minuten und insbesondere 60 bis 180 Minuten.

Bei den in Schritt b) in den Wäschebehandlungsraum eingebrachten Textilien kann es sich beispielsweise um Baumwoll- oder Synthetiktextilien aber auch um Mischgewebe handeln.

In einer bevorzugten Ausführungsform des Waschverfahrens verfügt die Trommelwaschmaschine über eine Vorrichtung zur Erfassung des Gewichts der in Schritt b) eingebrachten Textilien.

In einer beispielhaften Ausführungsform verfügt die Trommelwaschmaschine als Vorrichtung zur Erfassung der Textilgewichts über einen Beladungssensor. Der Einsatz eines solchen Sensors ermöglicht die direkte Ermittlung des Gewichts der in Schritt b) in den Wäschebehandlungsraum der Waschmaschine eingebrachten Textilien.

Vorzugsweise ist das Waschprogramm derart ausgestaltet, dass das mittels der Vorrichtung erfasste Textilgewicht die Menge der in Schritt e) eingebrachten zweiten Waschmittelzusammensetzung beeinflusst.

Die in Schritt c) in den Wäschebehandlungsraum eingebrachte wässrige Flotte weist vorzugsweise ein Volumen von 3 bis 40 I, vorzugsweise von 6 bis 30 I und insbesondere 8 bis 20 l auf.

Da das erfindungsgemäße Waschverfahren insbesondere bei hohen Wasserhärten überdurchschnittliche Reinigungsergebnisse erzielt, beträgt die Wasserhärte der in Schritt c) eingebrachten wässrigen Flotte vorzugsweise mehr als 14°dH, bevorzugt mehr als 17°dH und insbesondere mehr als 21°dH.

In einer bevorzugten Ausführungsform des Waschverfahrens verfügt die Trommelwaschmaschine über eine Vorrichtung zur Erfassung der Wasserhärte der in Schritt c) eingesetzten wässrigen Flotte.

In einer beispielhaften Ausführungsform verfügt die Trommelwaschmaschine als Vorrichtung zur Erfassung der Wasserhärte über einen Sensor zur Bestimmung der Wasserhärte. Der Einsatz eines solchen Sensors ermöglicht die direkte Ermittlung der Härte der in Schritt c) in den Wäschebehandlungsraum der Waschmaschine eingebrachten wässrigen Flotte.

In einer alternativen Ausführungsform wird die Härte der in Schritt c) in den Wäschebehandlungsraum der Waschmaschine eingebrachten wässrigen Flotte in indirekter Weise ermittelt. Einen indirekten Zugang zur Wasserhärte bietet beispielweise die Ermittlung des geografischen Standorts der Trommelwaschmaschine und Abgleich dieser Standortdaten mit den in einer Datenbank für unterschiedliche Standorte hinterlegte Wasserhärten. Die Standortdaten können durch den Nutzer beispielsweise in Form von Koordinaten oder den Koordinaten äquivalenten Informationen wie Postleitzahlen eingegeben werden oder automatisch durch die Waschmaschine selbst, beispielsweise durch ein GPS-Ortungssystem, bestimmt werden.

Vorzugsweise ist das Waschprogramm derart ausgestaltet, dass die mittels der Vorrichtung erfassten Wasserhärten die Menge der in Schritt e) eingebrachten zweiten Waschmittelzusammensetzung beeinflusst.

Die wässrige Flotte weist in Schritt c) vorzugsweise eine Temperatur T₁ von 18 bis 25°C auf.

Die Beladung der Waschmaschine mit Textilien in Schritt b) und das Volumen der in Schritt c) eingebrachten wässrigen Flotte werden vorzugsweise derart aufeinander abgestimmt, dass das Gewichtsverhältnis von wässriger Flotte zu Textilien in Schritt c) oberhalb 1:1, vorzugsweise oberhalb 2:1 und insbesondere oberhalb 5:2 liegt.

Die erste Waschmittelzusammensetzung wird in Schritt d) zu einem Zeitpunkt 0 bis 10% t_{w} in den Wäschebehandlungsraum eingebracht. Mit anderen Worten befindet sich die erste Waschmittelzusammensetzung bereits zu Beginn des Hauptwaschgangs in dem Wäschebehandlungsraum (Zeitpunkt 0 t_{w}) oder wird innerhalb eines Zeitraums von 10% der Dauer t_{w} des Hauptwaschgangs in den Wäschebehandlungsraum eingebracht.

In einer ersten Verfahrensvariante wird die wässrige Flotte vor der ersten Waschmittelzusammensetzung in den Wäschebehandlungsraum der Waschmaschine eingebracht. Eine solche Verfahrensvariante ist beispielsweise mittels eines Vorspülgangs realisierbar, in dessen Verlauf die Textilien mit Wasser vorgespült und/oder eingeweicht jedoch noch nicht mittels der eigentlichen Waschmittelzusammensetzung gereinigt werden.

Eine zweite Verfahrensvariante sieht den gleichzeitigen Eintrag der wässrigen Flotte und der ersten Waschmittelzusammensetzung in den Wäschebehandlungsraum der Waschmaschine vor. Wird die wässrige Flotte bei Verzicht auf einen Vorspülgang beispielsweise durch die mit der ersten Waschmittelzusammensetzung befüllten Einspülschublade der Waschmaschine in den Wäschebehandlungsraum geleitet, so erfolgt der Eintrag von wässriger Flotte und erster Waschmittelzusammensetzung in die Wäschebehandlungsraum gleichzeitig.

Schließlich ist es auch möglich, dass die wässrige Flotte nach der ersten Waschmittelzusammensetzung in den Wäschebehandlungsraum der Waschmaschine eingebracht wird, beispielsweise indem die erste Waschmittelzusammensetzung in vorportionierter Form oder mittels einer Dosierhilfe vor Beginn des Waschprogramms direkt auf die Textilien aufgebracht wird.

Die in Schritt d) eingesetzte Tensid-haltige erste Waschmittelzusammensetzung ist wie in Anspruch 1 definiert.

Die in Schritt d) eingesetzte Tensid-haltige ersten Waschmittelzusammensetzung wird vorzugsweise aus einem in die Waschmaschine integrierten Behälter dosiert, welcher mit der mehrfachen des für die Durchführung eines Waschprogramms notwendigen Menge der ersten Waschmittelzusammensetzung befüllt ist.

Zur Gruppe der Tenside werden die nichtionischen, die anionischen, die kationischen und die amphoteren Tenside gezählt. Die erfindungsgemäßen Zusammensetzungen enthalten Tensid aus der Gruppe der anionischen und nichtionischen Tenside.

Das anionische Tensid ist bevorzugt ausgewählt aus der Gruppe umfassend C₉₋₁₃-Alkylbenzolsulfonsäuren, Alkylethersulfonsäuren und Fettsäuren.

Zusammensetzungen, die als anionisches Tensid C₉₋₁₃-Alkylbenzolsulfonsäuren und Fettalkoholethersulfonsäuren umfassen, weisen besonders gute, dispergierende Eigenschaften auf.

In besonders bevorzugten Waschverfahren enthält die ersten Waschmittelzusammensetzung anionisches Tensid aus der Gruppe aus der Gruppe der C₈₋₁₈-Alkylbenzolsulfonsäuren. Besonders bevorzugt ist der Einsatz von Alkylbenzolsulfonsäuren aus der Gruppe der C₉₋₁₅-Alkylbenzolsulfonsäuren, insbesondere der C₉₋₁₃-Alkylbenzolsulfonsäuren.

Alternativ, vorzugsweise jedoch in Kombination mit der Alkylbenzolsulfonsäure umfasst die ersten Waschmittelzusammensetzung anionisches Tensid aus der Gruppe aus der Gruppe der Alkylethersulfonsäuren.

Bevorzugt sind Alkylethersulfonsäuren mit der Formel R₁-O-(AO)ₙ-SO₃H, wobei
- R₁ für einen linearen oder verzweigten, substituierten oder unsubstituierten Alkylrest, vorzugsweise für einen linearen, unsubstituierten Alkylrest, besonders bevorzugt für einen Fettalkoholrest steht. Bevorzugte Reste R₁ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R₁ sind abgeleitet von Fettalkoholen mit 12 bis 18 C-Atomen, beispielsweise von Kokosfettalkohol, Talgfettalkohol, Lauryl-, Myristyl-, Cetyl- oder Stearylalkohol oder von Oxoalkoholen mit 10 bis 20 C-Atomen.
- AO für eine Ethylenoxid- (EO) oder Propylenoxid- (PO) Gruppierung, vorzugsweise für eine Ethylenoxidgruppierung steht. Der Index n der Formel ist eine ganze Zahl von 1 bis 50, vorzugsweise von 1 bis 20 und insbesondere von 2 bis 10. Ganz besonders bevorzugt ist n 2, 3, 4, 5, 6, 7 oder 8.

Als weiteren optionalen Bestandteil, welcher in der ersten Waschmittelzusammensetzung vorzugsweise in Kombination mit den vorgenannten Alkylbenzolsulfonsäuren und Alkylethersulfonsäuren vorliegt, enthält das die erste Waschmittelzusammensetzung anionisches Tensid aus der Gruppe aus der Gruppe der Fettsäuren. Besonders bevorzugte Fettsäuren sind ausgewählt aus der Gruppe Caprylsäure, Caprinsäure, Laurinsäure, Myristinsäure, Palmitinsäure, Stearinsäure, Ölsäure, Linolsäure und deren Mischungen.

Erfindungsgemäss in Schritt d) des Verfahrens eingesetzte erste Waschmittelzusammensetzungen enthalten, bezogen auf ihr Gesamtgewicht, 40 bis 80 Gew.-% und insbesondere 45 bis 70 Gew.-% anionisches Tensid. Der Gewichtsanteil des anionischen Tensids bestimmt sich unabhängig vom Neutralisationsgrad des Aniontensid in der ersten Waschmittelzusammensetzung als der Gewichtsanteil der Aniontensidsäure.

Als weiteren Bestandteil enthält die erste Waschmittelzusammensetzung nichtionisches Tensid aus der Gruppe der ethoxylierten primären C6-18-Alkohole, vorzugsweise der ethoxylierten primären C6-18-Alkohole mit einem Alkoxylierungsgrad ≥ 2, besonders bevorzugt der C12-14-Alkohole mit 4 EO oder 7 EO, der C9-11-Alkohole mit 7 EO, der C13-15-Alkohole mit 5 EO, 7 EO oder 8 EO, der C13-15-Oxoalkohole mit 7 EO, der C12-18-Alkohole mit 5 EO oder 7 EO, insbesondere der C12-18-Fettalkohole mit 7 EO oder der C13-15-Oxoalkohole mit 7 EO.

Erfindungsgemäss in Schritt d) des Verfahrens eingesetzte erste Waschmittelzusammensetzungen enthalten, bezogen auf ihr Gesamtgewicht, 12 bis 40 Gew.-% und insbesondere 15 bis 30 Gew.-% nichtionisches Tensid.

Eine Gruppe optionaler Bestandteile der ersten Waschmittelzusammensetzung bilden die polyalkoxylierte Amine mit einem gewichtsmittleren Molekulargewicht Mw im Bereich von 600 g/mol bis 10000 g/mol, welche erhältlich sind durch Umsetzung von Ammoniak oder primären Alkyl- oder Hydroxyalkylaminen, die ein Molekulargewicht unter 200 g/mol aufweisen, mit Alkylenoxiden. Der Gewichtsanteil dieser polylalkoxylierten Amine am Gesamtgewicht der ersten Waschmittelzusammensetzung beträgt vorzugsweise 0,5 bis 10 Gew.-%, besonders bevorzugt 1 bis 8 Gew.-% und insbesondere 2 bis 6 Gew.-%.

Bevorzugte polyalkoxylierte Amine weisen ein gewichtsmittleres Molekulargewicht M_{w} im Bereich von 1300 g/mol bis 6000 g/mol, insbesondere von 1400 g/mol bis 4500 g/mol auf. (Bei den hier und später gegebenenfalls für andere Polymere angegebenen mittleren Molekulargewichten handelt es sich um gewichtsmittlere Molekulargewichte M_{w}, die grundsätzlich mittels Gelpermeationschromatographie mit Hilfe eines RI-Detektors bestimmbar sind, wobei die Messung zweckmäßig gegen einen externen Standard erfolgt.) Zu ihrer Herstellung kann man in bekannter Wiese von Ammoniak, einem Monoalkylamin, einem Monoalkyl-monoalkanolamin oder einem Monoalkyl-dialkanolamin oder einem Mono-, Di- oder Trialkanolamin, beispielsweise Triethanolamin, Methyl-, Ethyl-, Propyl- und Isopropyldiethanolamin, Methyl-, Ethyl-, Propyl- und Isopropyl-diisopropanolamin, Tripropanolamin, Triisopropanolamin, N,N-Di-(2-hydroxyethyl)cyclohexylamin, N,N-Di-(2-hydroxypropyl)cyclohexylamin, n-Butylamin, n-Hexylamin, n-Octylamin, Isopropylamin, sek-Butylamin, tert-Butylamin, Cyclohexylamin, 2-Ethylhexylamin, 2-Phenylethylamin und deren Mischungen, ausgehen, das mit einem Alkylenoxid, insbesondere ausgewählt aus der Gruppe bestehend aus Ethylenoxid, Propylenoxid, Butylenoxid und Mischungen daraus, umgesetzt wird, insbesondere mit einer Mischung enthaltend Propylenoxid und vorzugsweise Ethylenoxid, besonders bevorzugt mit Propylenoxid. Bei den so erhältlichen polyalkoxylierten Aminen kann es sich um Block- oder Random-Strukturen handeln. Besonders bevorzugt ist unter anderem ein polyalkoxyliertes Amin, erhältlich durch Propoxylierung von Triethanolamin, bevorzugt mit einer Länge der drei Seitenarme von jeweils 15 Propylenoxid-Einheiten. Ebenfalls bevorzugt ist auch ein polyalkoxyliertes Amin, erhältlich durch Propoxylierung von Triisopropanolamin, bevorzugt mit einer Länge der drei Seitenarme von jeweils 15 Propylenoxid-Einheiten. Ebenfalls geeignet sind polyalkoxylierte Monoalkylamine mit einer linearen, verzweigten oder cyclischen Alkylgruppe, wobei mit einem Alkylenoxid ausgewählt aus der Gruppe bestehend aus Ethylenoxid, Propylenoxid, Butylenoxid und Mischungen daraus alkoxyliert wird, bevorzugt mit einer Mischung enthaltend Propylenoxid, besonders bevorzugt mit Propylenoxid. Bevorzugt ist auch ein polyalkoxyliertes Amin, erhältlich durch Propoxylierung von tert-Butylamin, bevorzugt mit einer Länge der zwei Seitenarme von jeweils 12 Propylenoxid-Einheiten.

Bevorzugte polyalkoxylierte Amine genügen der allgemeinen Formel, 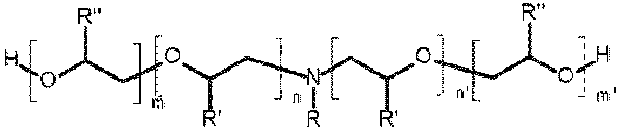
in der R für eine lineare, gegebenenfalls verzweigte oder gegebenenfalls cyclische Alkylgruppe mit 1 bis 12 C-Atomen oder einer Gruppe -(CH₂CHR'O)_{n"}-(CH₂CHR"O)_{m"}-H steht,
R' und R" unabhängig voneinander für H, CH₃ oder CH₂CH₃ stehen,
n, n' und n" unabhängig voneinander für Zahlen von 0 bis 30, vorzugsweise von 0 bis 10 und insbesondere 0 bis 5 stehen, und
m, m' und m" unabhängig voneinander für Zahlen von 0 bis 30, vorzugsweise von 5 bis 20 und insbesondere von 12 bis 16 stehen,
mit der Maßgabe, dass die Summe n + n' + n" + m + m' + m" mindestens 14 ist, vorzugsweise im Bereich von 18 bis 100 und insbesondere im Bereich von 20 bis 70 liegt. Bevorzugt ist in den Verbindungen der Formel I mindestens einer der Reste R' und R" eine CH₃-Gruppe.

Als weiteren optionalen Bestandteil enthält die erste Waschmittelzusammensetzung ein polyalkoxyliertes Polyalkylenimin, das erhältlich ist durch Umsetzung von Polyalkyleniminen mit Alkylenoxiden, wobei Gewichtsanteile am Gesamtgewicht der ersten Waschmittelzusammensetzung von 0,5 bis 10 Gew.-%, vorzugsweise von 1 bis 8 Gew.-% und insbesondere von 2 bis 6 Gew.-% bevorzugt sind.

Bei dem polyalkoxylierten Polyalkylenimin handelt es sich um ein Polymer mit einem Polyalkylenimin-Rückgrat, das an den N-Atomen Polyalkoxygruppen trägt. Es weist vorzugsweise ein gewichtsmittleres Molekulargewicht Mw im Bereich von 5000 g/mol bis 60000 g/mol, insbesondere von 10000 g/mol bis 22500 g/mol auf. Das Polyalkylenimin weist an den Enden primäre Aminofunktionen und im Inneren vorzugsweise sowohl sekundäre als auch tertiäre Aminofunktionen auf; gegebenenfalls kann es im Inneren auch lediglich sekundäre Aminofunktionen aufweisen, so dass sich nicht ein verzweigtkettiges, sondern ein lineares Polyalkylenimin ergibt. Das Verhältnis von primären zu sekundären Aminogruppen im Polyalkylenimin liegt vorzugsweise im Bereich von 1:0,5 bis 1:1,5, insbesondere im Bereich von 1:0,7 bis 1:1. Das Verhältnis von primären zu tertiären Aminogruppen im Polyalkylenimin liegt vorzugsweise im Bereich von 1:0,2 bis 1:1, insbesondere im Bereich von 1:0,5 bis 1:0,8. Vorzugsweise weist das Polyalkylenimin ein gewichtsmittleres Molekulargewicht im Bereich von 500 g/mol bis 50000 g/mol, insbesondere von 550 g/mol bis 2000 g/mol auf. Die N-Atome im Polyalkylenimin sind vorzugsweise durch Alkylengruppen mit 2 bis 12 C-Atomen, insbesondere 2 bis 6 C-Atomen, voneinander getrennt, wobei nicht sämtliche Alkylengruppen die gleiche C-Atomanzahl aufweisen müssen. Besonders bevorzugt sind Ethylengruppen, 1,2-Propylengruppen, 1,3-Propylengruppen, und deren Mischungen. Die primären Aminofunktionen im Polyalkylenimin können 1 oder 2 Polyalkoxygruppen und die sekundären Aminofunktionen 1 Polyalkoxygruppe tragen, wobei nicht jede Aminofunktion alkoxygruppensubstituiert sein muss. Die durchschnittliche Anzahl von Alkoxygruppen pro primärer und sekundärer Aminofunktion im polyalkoxylierten Polyalkyenimin beträgt vorzugsweise 5 bis 100, insbesondere 10 bis 50. Bei den Alkoxygruppen im polyalkoxylierten Polyalkylenimin handelt es sich vorzugsweise um Ethoxy-, Propoxy- oder Butoxygruppen oder Mischungen aus diesen. Besonders bevorzugt sind polyethoxylierte Polyethylenimine. Die polyalkoxylierten Polyalkylenimine sind durch Umsetzung der Polyalkylenimine mit den Alkoxygruppen entsprechenden Epoxiden zugänglich. Gewünschtenfalls kann die endständige OH-Funktion zumindest einiger der Polyalkoxysubstituenten durch eine Alkylether-Funktion mit 1 bis 10, insbesondere 1 bis 3 C-Atomen, ersetzt sein.

Für den Einsatz in dem erfindungsgemäßen Waschverfahren werden Waschmittelzusammensetzungen mit einem möglichst geringen Anteil nicht aktiver Inhaltsstoffe vorgesehen. Folglich ist es erfindungsgemäss, wenn die ersten Waschmittelzusammensetzung weniger als 6 Gew.-% und insbesondere weniger als 2 Gew.-% Wasser enthält. Mit sinkendem Anteil nicht aktiver Inhaltsstoffe nimmt nicht nur der Umfang der für die Verpackung, Lagerung und Transport der ersten Waschmittelzusammensetzung notwendigen Ressourcen ab, gleichzeitig nimmt auch das Volumen der pro Waschverfahrens eingesetzten ersten Waschmittelzusammensetzung ab und damit die Reichweite eines Vorratsbehälters, beispielsweise eines in die Waschmaschine integrierten Vorratsbehälters zu.

Für die Handhabbarkeit der ersten Waschmittelzusammensetzung, insbesondere deren Dosierung hat sich ein, auf das Gesamtgewicht der ersten Waschmittelzusammensetzung bezogener Gewichtsanteil 4,5 bis 27 Gew.-% und insbesondere 10 bis 24 Gew.-% organischen Lösungsmittels als vorteilhaft erwiesen. Organischen Lösungsmitteln können im Waschverfahren reinigungsverstärkende Wirkungen zugerechnet werden. Dies gilt insbesondere bei Einsatz organischer Lösungsmittel aus der Gruppe der organischen Amine, vorzugsweise Monoethanolamin. Der Einsatz dieser hat nicht nur eine reinigungsverstärkende Wirkung, sondern ermöglicht zudem die partielle oder vollständige Neutralisation etwaiger in der ersten Waschmittelzusammensetzung enthaltener anionischer Tensidsäuren.

In einer bevorzugten Ausführungsform des Waschverfahrens umfasst die erste Waschmittelzusammensetzung anionisches Tensid aus der Gruppe der C₈₋₁₈-Alkylbenzolsulfonsäuren, der Alkylethersulfonsäuren und der Fettsäuren sowie eine in Bezug auf das anionische Tensid überstöchiometrische Menge organisches Amin, vorzugsweise Monoethanolamin.

Zusammenfassend sind Waschverfahren erfindungsgemäss, wenn die erste Waschmittelzusammensetzung in Schritt d), bezogen auf ihr Gesamtgewicht
i) 40 bis 80 Gew.-% und insbesondere 45 bis 70 Gew.-% anionisches Tensid aus der Gruppe der C₈₋₁₈-Alkylbenzolsulfonsäuren, der Alkylethersulfonsäuren und der Fettsäuren,
ii) 12 bis 40 Gew.-% und insbesondere 15 bis 30 Gew.-% nichtionisches Tensid aus der Gruppe der ethoxylierten primären C6-18-Alkohole,
iii) 4,5 bis 27 Gew.-% und insbesondere 10 bis 24 Gew.-% organisches Lösungsmittel aus der Gruppe der organischen Amine
iv) weniger als 6 Gew.-% und insbesondere weniger als 2 Gew.-% Wasser enthält.

Die erste Waschmittelzusammensetzung wird in einer Verfahrensvariante in Schritt d) vorzugsweise zu einem Zeitpunkt 0 t_{w} in den Wäschebehandlungsraum der Waschmaschine eingebracht.

Mit größerem Vorzug wird die erste Waschmittelzusammensetzung in Schritt d) zu einem Zeitpunkt >0 bis 10% t_{w}, vorzugsweise >0 bis 5% t_{w} in den Wäschebehandlungsraum der Waschmaschine eingebracht.

Dem gegenüber wird die zweite Waschmittelzusammensetzung in Schritt e) zu einem Zeitpunkt 11 bis 99% t_{w}, vorzugsweise zu einem Zeitpunkt 25 bis 65% t_{w}, besonders bevorzugt zu einem Zeitpunkt 30 bis 60% t_{w} in die wässrige Flotte eingebracht.

Die zweite Waschmittelzusammensetzung ist im Hinblick auf ihren Gehalt an aktivem Protease Enzym hochkonzentriert und umfasst bezogen auf ihr Gesamtgewicht, mindestens 4 Gew.-% aktives Protease-Protein sowie mindestens 80 Gew.-% eines flüssigen Trägers

Proteasen gehören zu den technisch bedeutendsten Enzymen. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Subtilasen werden natürlicherweise von Mikroorganismen gebildet. Hierunter sind insbesondere die von *Bacillus*-Spezies gebildeten und sezernierten Subtilisine als bedeutendste Gruppe innerhalb der Subtilasen zu erwähnen.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die alkalische Protease aus *Bacillus lentus*, insbesondere aus *Bacillus lentus* DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so beispielsweise für den Einsatz in Wasch- und Reinigungsmitteln optimiert. Dazu gehören Punkt-, Deletions- oder Insertionsmutagenese oder Fusion mit anderen Proteinen oder Proteinteilen. So sind für die meisten aus dem Stand der Technik bekannten Proteasen entsprechend optimierte Varianten bekannt.

In bevorzugten Ausführungsformen des Verfahrens umfasst die zweite Waschmittelzusammensetzung, bezogen auf ihr Gesamtgewicht, 4 bis 18 Gew.-%, vorzugsweise 5 bis 15 Gew.-% und insbesondere 7 bis 12 Gew.-% aktives Protease-Protein. Die Konzentration an aktivem Protease-Protein in der zweiten Waschmittelzusammensetzung liegt damit um den Faktor 10 bis 40 oberhalb der Konzentration von aktivem Protease-Protein in üblichen kommerziell erhältlichen Waschmittelzusammensetzungen.

### Bevorzugte Proteasen umfassen

a) eine Protease, die proteolytische Aktivität aufweist und eine Aminosäuresequenz umfasst, die zu der in SEQ ID NO:1 [BLAP] angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90,5%, 91%, 91,5%, 92%, 92,5%, 93%, 93,5%, 94%, 94,5%, 95,5%, 96%, 96,5%, 97%, 97,5%, 98%, 98,5% oder 98,8% identisch ist und, jeweils bezogen auf die Nummerierung gemäß SEQ ID NO:3 [BPN'], (i) an der Position, die der Position 101 entspricht, die Aminosäuresubstitution R101E, und (ii) an den mindestens einer der Positionen, die den Positionen 3, 4, 45, 55, 58, 59, 61, 87, 97, 98, 106, 117, 120, 124, 129, 136, 137, 143, 156, 161, 163, 171, 172, 185, 199, 205, 209, 222, 238, 244, 261 und 262 entsprechen, mindestens eine Aminosäuresubstitution, die aus der aus S3T, V4I, R45E, R45D, R45Q, P55N, T58W, T58Y, T58L, Q59D, Q59M, Q59N, Q59T, G61D, G61R, S87E, G97S, A98D, A98E, A98R, S106A, S106W, N117E, H120V, H120D, H120K, H120N, S124M, P129D, E136Q, Q137H, S143W, S156D, S161T, S163A, S163G, Y171L, A172S, N185Q, V199M, V205I, Y209W, M222Q, N238H, V244T, N261T und L262N, L262Q, L262D, L262E bestehenden Gruppe ausgewählt ist, aufweist; [→THOR / HET und VINZON; umfasst aber durch 70% seq id auch weitere HET Varianten, wie z.B. HEY], wobei die Aminosäuresubstitutionskombination der Gruppe (ii) vorzugsweise aus der aus N238E-L262E, S156D-L262E [= Vinzon], S3T-V4I-V205I [= HET], S3T-V4I-A228V, G195E-V199M, H120D-S163G-N261D, N76D-A228V-N261D, S3T-N76D-S156D-Y209W, Q137H-S141H-R145H-N238E-L262E, Q137H-S141H-R145H-S156D-L262E, N76D-Q137H-S141H-R145H-A228V-N261D, N76D-Q137H-S141H-R145H-S163G-N238E, H120D-Q137H-S141H-R145H-S163G-N261D, S3T-N76D-Q137H-S141H-R145H-S156D-Y209W bestehenden Gruppe ausgewählt ist;
b) eine Protease, die proteolytische Aktivität aufweist und eine Aminosäuresequenz umfasst, die zu der in SEQ ID NO:2 [HP388] angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5% und 98% identisch ist und, jeweils bezogen auf die Nummerierung gemäß SEQ ID NO:3, (i) an den Positionen, die den Positionen 9, 130, 133, 144, 217, 252 und 271 entsprechen, mindestens eine Aminosäuresubstitution, die aus der aus P9T, N130D, N130V, T133A, N144K, Y217M, N252T und Q271E bestehenden Gruppe ausgewählt ist, und (ii) an mindestens einer der Positionen, die den Positionen 6, 61, 62, 63, 89, 99, 101, 131, 156, 166, 170, 187, 188, 189, 211 und 224 entsprechen, mindestens eine Aminosäuresubstitution, die aus der aus Y6F, Y6W, F61G, Q62N, S63Q, S89A, S89G, N99H, D101S, D101E, D101A, G131H, G131Y, G131F, S156R, G166A, G166M, G166L, G166I, K170R, K170G, N187D, N188G, S189T, S189L, S189I, S189R, S211N, S211Q, S224A, S224G bestehenden Gruppe ausgewählt ist, aufweist [→ HP545 und Varianten davon → als Alternative zu HEY, Vinzon etc. im Fokus], wobei die Protease vorzugsweise eine Aminosäuresubstitutionskombination aufweist, die aus der aus P9T-N130D-T133A-N144KG166M-S189T-Y217M-N252T-Q271E, P9T-N130D-T133A-N144K-G166M-S189T-Y217M-S224AN252T-Q271E, P9T-N130D-T133A-N144K-G166M-S211N-Y217M-N252T-Q271E, P9T-S89A-N130D-G131H-T133A-N144K-S189T-Y217M-S224A-N252T-Q271E, P9T-S89A-N130D-T133A-N144K-S189T-Y217M-S224A-N252T-Q271E [= HP545], P9T-S89A-N130D-T133A-N144K-S189T-Y217M-S224A-N252T-Q271E, P9T-S89A-N130D-T133A-N144K-N187D-Y217M-S224A-N252T-Q271E, P9T-S89A-N130D-T133A-N144K-S189R-Y217M-S224A-N252T-Q271E, P9T-S89A-N130D-T133A-N144K-N187D-S189R-Y217M-S224A-N252T-Q271E, P9T-S89A-D101S-N130D-T133A-N144K-S189T-Y217M-S224A-N252T-Q271E, P9T-S89A-D101E-N130D-T133A-N144K-S189T-Y217M-S224AN252T-Q271E, P9T-S89A-D101A-N130D-T133A-N144K-S189T-Y217M-S224A-N252T-Q271E, P9T-S89A-N99H-N130D-T133A-N144K-K170R-S189T-Y217M-S224A-N252T-Q271E, P9T-S89A-N130D-T133A-N144K-S156R-S189T-Y217M-S224A-N252T-Q271E, P9T-S63Q-S89A-N130D-T133A-N144KG166A-S189T-Y217M-S224A-N252T-Q271E, P9T-F61G-Q62-N-S89A-N130D-T133A-N144K-N188G-S189T-Y217M-S224A-N252T-Q271E, Y6W-P9T-N130D-T133A-N144K-S189T-Y217M-S224A-N252T-Q271E, Y6W-P9T-S89A-N130D-T133A-N144K-S189T-Y217M-S224A-N252T-Q271E, Y6W-P9T-F61G-Q62N-S89A-N130D-T133A-N144K-S189T-Y217M-S224A-N252T-Q271E, Y6W-P9T-S89AN130D-T133A-N144K-N188G-S189T-Y217M-S224A-N252T-Q271E bestehenden Gruppe ausgewählt ist;
c) eine Protease, die proteolytische Aktivität aufweist und eine Aminosäuresequenz umfasst, die zu der in SEQ ID NO:3 [BLAP] angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90,5%, 91%, 91,5%, 92%, 92,5%, 93%, 93,5%, 94%, 94,5%, 95,5%, 96%, 96,5%, 97%, 97,5%, 98%, 98,5% oder 98,8% identisch ist und, jeweils bezogen auf die Nummerierung gemäß SEQ ID NO:3 (i) an den Positionen, die den Positionen 3, 4, 101 und 205 entsprechen, die Aminosäuresubstitutionen S3T, V4I, R101E und V205I, und (ii) an mindestens einer der Positionen, die den Positionen 76, 138, 145, 156, 166, 167, 169, 177, 187, 189, 191, 206, 209, 215, 218 oder 262 entsprechen, mindestens eine Aminosäuresubstitution, die aus der aus N76D, N76E, N76Q, A138Q, R145L, R145W, R145Y, S156D, S156Q, S166G, Y167T, A169G, V177L, A187D, F189R, Q191R, Q206A, Q206L, Q206S, Q206T, Y209K, Y209V, Y209W, A215K, A215W, N218S, N218T und L262D, L262E und L262Q bestehenden Gruppe ausgewählt ist, aufweist, wobei die Protease vorzugsweise eine Aminosäuresubstitutionskombination aufweist, die aus der aus S3T-V4I-R101E-V205I-Q206L-Y209W, S3T-V4I-R101E-V205I-N218S, S3T-V4I-R101E-V205I-N76D, S3T-V4I-R101E-V205I-S156D-L262E, S3T-V4I-R101E-V205I-Q206L-Y209W-S156D-L262E, S3T-V4I-R101E-V205I-N76D-Q206L-Y209W, S3T-V4I-R101E-V205I-N76D-S156D-Q206L-Y209W-L262E, S3T-V4I-R101E-V205I-N76D-N218S, S3T-V4I-R101E-V205I-N76D-S156D-Y209W-L262E, S3T-V4I-R101E-V205I-N76D-Y209W, S3T-V4I-R101E-V205I-N76D-S156D-Q206L-L262E, S3T-V4I-R101E-V205I-N76D-Q206L, S3T-V4I-R101E-V205I-S156D-Q206L-Y209W, S3T-V4I-R101E-V205I-Q206L-Y209W-L262E, S3T-V4I-R101E-V205I-A138Q-R145W-Y167T-Q206L, S3T-V4I-R101E-V205I-N76D-R145Y-A215W-N218S-L262E, S3T-V4I-R101E-V205I-N76D-S156D-Y209W-L262E; S3T-V4I-R101E-V205I-Q206L-Y209W-A215K-S156D-L262E [=HEY], S3T-V4I-R101E-V205I-S156D-S166G-Q191R-Q206L-Y209W-L262E, S3T-V4I-R101E-V205I-S156D-A187D-F189R-Q206L-Y209W-L262E und S3T-V4I-R101E-V205I-A138Q-S156D-V171L-Q206L bestehenden Gruppe ausgewählt ist.

Im Hinblick auf die erzielte Reinigungsleistung, insbesondere auch bei hohen Wasserhärten oder hoher Beladung, hat es sich als vorteilhaft erwiesen, wenn das aktive Protease-Protein proteolytische Aktivität aufweist und eine Aminosäuresequenz umfasst, die mindestens 70% Sequenzidentität mit der in SEQ ID NO:1 angegebenen Aminosäuresequenz über deren Gesamtlänge aufweist und jeweils bezogen auf die Nummerierung gemäß SEQ ID NO:3 (i) an mindestens zwei, vorzugsweise drei der Positionen, die den Positionen 3, 4, 101 oder 205 entsprechen, mindestens zwei Aminosäuresubstitutionen, vorzugsweise drei Aminosäuresubstitutionen, ausgewählt aus den Aminosäuresubstitutionen S3T, V4I, R101E und V205I, und (ii) an mindestens einer der Positionen, die den Positionen 76, 138, 145, 156, 166, 167, 169, 177, 187, 189, 191, 206, 209, 215, 218 oder 262 entsprechen, mindestens eine weitere Aminosäuresubstitution, insbesondere ausgewählt aus N76D, N76E, N76Q, A138Q, R145L, R145W, R145Y, S156D, S156Q, S166G, Y167T, A169G, V177L, A187D, F189R, Q191R, Q206A, Q206L, Q206S, Q206T, Y209K, Y209V, Y209W, A215K, A215W, N218S, N218T, L262D, L262E und L262Q, aufweist.

Die erfindungsgemäßen Proteasen weisen enzymatische Aktivität auf, d.h. sie sind zur Hydrolyse von Peptiden und Proteinen befähigt, insbesondere in einem Wasch- oder Reinigungsmittel. Eine erfindungsgemäße Protease ist daher ein Enzym, welches die Hydrolyse von Amid/Peptidbindungen in Protein/Peptid-Substraten katalysiert und dadurch in der Lage ist, Proteine oder Peptide zu spalten. Ferner handelt es sich bei einer erfindungsgemäßen Protease vorzugsweise um eine reife (mature) Protease, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

Verfahren zur Bestimmung der Protease-Aktivität dem Fachmann auf dem Gebiet der Enzymtechnologie geläufig und werden von ihm routinemäßig angewendet. Beispielsweise sind solche Verfahren offenbart in Tenside, Band 7 (1970), S. 125-132. Alternativ kann die Protease-Aktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20 s bis 60 s. Die Protease-Aktivität wird üblicherweise in Protease-Einheiten (PE) angegeben. Geeignete Protease-Aktivitäten betragen beispielsweise 2,25, 5 oder 10 PE pro ml Waschflotte. Die Protease-Aktivität ist jedoch nicht gleich Null.

Ein alternativer Test zur Feststellung der proteolytischen Aktivität der erfindungsgemäßen Proteasen ist ein optisches Messverfahren, bevorzugt ein photometrisches Verfahren. Der hierfür geeignete Test umfasst die Protease-abhängige Spaltung des Substratproteins Casein. Dieses wird durch die Protease in eine Vielzahl kleinerer Teilprodukte gespalten. Die Gesamtheit dieser Teilprodukte weist eine erhöhte Absorption bei 290 nm gegenüber nicht gespaltenem Casein auf, wobei diese erhöhte Absorption unter Verwendung eines Photometers ermittelt werden und somit ein Rückschluss auf die enzymatische Aktivität der Protease gezogen werden kann.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (Gornall et al., J. Biol. Chem. 177 (1948): 751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (vgl. Bender et al., J. Am. Chem. Soc. 88, 24 (1966): 5890-5913) erfolgen.

In verschiedenen Ausführungsformen der Erfindung ist die Protease ein frei vorliegendes Enzym. Dies bedeutet, dass die Protease mit allen Komponenten eines Mittels direkt agieren kann und, falls es sich bei dem Mittel um ein Flüssigmittel handelt, dass die Protease direkt mit dem Lösungsmittel des Mittels (z.B. Wasser) in Kontakt steht. In anderen Ausführungsformen kann ein Mittel Proteasen enthalten, die einen Interaktionskomplex mit anderen Molekülen bilden oder die eine "Umhüllung" enthalten. Hierbei kann ein einzelnes oder mehrere Protease Moleküle durch eine sie umgebende Struktur von den anderen Bestandteilen des Mittels getrennt sein. Eine solche trennende Struktur kann entstehen durch, ist allerdings nicht beschränkt auf, Vesikel, wie etwa eine Micelle oder ein Liposom. Die umgebende Struktur kann aber auch ein Viruspartikel, eine bakterielle Zelle oder eine eukaryotische Zelle sein. In verschiedenen Ausführungsformen kann ein Mittel Zellen von *Bacillus pumilus* oder *Bacillus subtilis,* die die erfindungsgemäßen Proteasen exprimieren, oder Zellkulturüberstände solcher Zellen enthalten.

Im Zusammenhang mit der vorliegenden Erfindung bedeutet das Merkmal, dass eine Protease die angegebenen Substitutionen aufweist, dass sie an der jeweiligen Position eine (der angegebenen) Substitution(en) enthält, d.h. zumindest die angegebenen Positionen nicht anderweitig mutiert oder, beispielsweise durch Fragmentierung der Protease, deletiert sind. In verschiedenen bevorzugten Ausführungsformen weisen die hierin beschriebenen Proteasen mit Ausnahme der explizit erwähnten Substitutionen die Sequenz von SEQ ID NO:1 auf, d.h. sind abgesehen von den substituierten Positionen 100% identisch zu der Sequenz gemäß SEQ ID NO:1.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. z.B. Altschul et al. (1990) Basic local alignment search tool, J. Mol. Biol., 215:403-410, und Altschul et al. (1997) Gapped BLAST and PSI-BLAST: a new generation of protein database search programs, Nucleic Acids Res., 25:3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. z.B. Chenna et al. (2003) Multiple sequence alignment with the Clustal series of programs, Nucleic Acid Res., 31:3497-3500), T-Coffee (vgl. z.B. Notredame et al. (2000) T-Coffee: A novel method for multiple sequence alignments, J. Mol. Biol., 302:205-217) oder Programme, die auf diesen Programmen bzw. Algorithmen basieren. Ferner möglich sind Sequenzvergleiche (Alignments) mit dem Computer-Programm Vector NTI^{®} Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert, oder Clone Manager 10 (Verwendung der Scoring Matrix BLOSUM 62 für Sequenz-Alignment auf Aminosäureebene). Soweit nicht anders angegeben, wird die hierin angegebene Sequenzidentität mit dem BLAST-Algorithmus bestimmt.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essenzielle Funktionen aus. Es kann daher sinnvoll sein, Sequenzübereinstimmungen nur auf einzelne, ggf. kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO:1 in einem wie oben definierten Alignment zugeordnet ist. Weiterhin richtet sich die Zuordnung der Positionen nach dem reifen (maturen) Protein. Diese Zuordnung ist insbesondere auch anzuwenden, wenn die Aminosäuresequenz einer erfindungsgemäßen Protease eine höhere Zahl von Aminosäureresten umfasst als die Protease gemäß SEQ ID NO:1. Ausgehend von den genannten Positionen in der Aminosäuresequenz der Protease gemäß SEQ ID NO:1 sind die Veränderungspositionen in einer erfindungsgemäßen Protease diejenigen, die eben diesen Positionen in einem Alignment zugeordnet sind. Die Nummerierung etwaiger Sequenzänderungen richtet sich, sofern nichts anderes angegeben ist, in der vorliegenden Anmeldungen nach der Nummerierung gemäß SEQ ID NO:3.

Zusätzlich zu den vorstehend erläuterten Aminosäureveränderungen können erfindungsgemäße Proteasen weitere Aminosäureveränderungen, insbesondere Aminosäure-Substitutionen, -Insertionen oder -Deletionen, aufweisen. Solche Proteasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Proteasen oder anderer Polypeptide genutzt werden. Das Ziel ist es, in die bekannten Moleküle gezielte Mutationen wie Substitutionen, Insertionen oder Deletionen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität oder katalytische Aktivität der Protease erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierter Proteasen, zum Beispiel hinsichtlich ihrer Stabilität bei Lagerung, kann daher im Rahmen der Erfindung zusätzlich weiterentwickelt sein.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere oder alternative Austausche innerhalb derselben Polypeptidkette werden durch Schrägstriche voneinander getrennt. "130D/V" bedeutet somit, dass die Position 130 zu D oder V mutiert ist. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt oder vor der entsprechenden Position ein Δ angegeben. Beispielsweise beschreibt P9T die Substitution von Prolin an Position 9 durch Threonin, P9TH die Insertion von Histidin nach der Aminosäure Threonin an Position 9 und P9* oder ΔP9 die Deletion von Prolin an Position 9. Diese Nomenklatur ist dem Fachmann auf dem Gebiet der Enzymtechnologie bekannt.

Neben dem aktiven Protease-Protein umfasst die zweite Waschmittelzusammensetzung als wesentlichen weiteren Bestandteil einen flüssigen Träger. Der Gewichtsanteil des flüssigen Trägers am Gesamtgewicht der zweiten Waschmittelzusammensetzung beträgt, bezogen auf deren Gesamtgewicht, 80 bis 96 Gew.-%, vorzugsweise 85 bis 95 Gew.-% und insbesondere 85 bis 90 Gew.-%.

Der wässrige Träger der zweiten Waschmittelzusammensetzung umfasst vorzugsweise ein wässrig-organisches Lösungsmittelgemisch. Besonders bevorzugt ist der Einsatz von organischem Lösungsmittel aus der Gruppe Ethanol, n-Propanol, i-Propanol, Butanolen, Glykol, Propandiol, Butandiol, Methylpropandiol, Glycerin, Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropy-lenglykolmonoethylether, Methoxytriglykol, Ethoxytriglykol, Butoxytriglykol, 1-Butoxyethoxy-2-propanol, 3-Methyl-3-methoxybutanol, Propylen-glykol-t-butylether, Di-n-octylether sowie deren Mischungen, insbesondere aus der Gruppe Propandiol, Glycerin und Ethanol.

Das aktive Protease-Protein kann in der zweiten Waschmittelzusammensetzung mit mindestens einer reversiblen Inhibitorverbindung, die aus der aus Polyolen, insbesondere Glycerin und 1,2-Propylenglykol, Benzamidinhydrochlorid, Borax, Borsäuren, Boronsäuren oder deren Salze oder Ester oder Derivate, insbesondere Phenylboronsäurederivate oder 4-Formylphenylboronsäure (4-FPBA), Verbindungen der Formeln (I) oder (II) 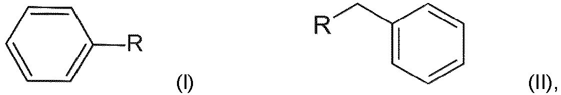 wobei R jeweils ausgewählt ist aus -COOH, C₁₋₆-Alkyl-substituierten oder unsubstituierten C₂₋₆-Dicarbonsäuren, C₁₋₆-Alkyl-substituierten oder unsubstituierten C₂₋₆-Carbonsäuren und -OOC-NR²₂, wobei R² gleich oder verschieden und ausgewählt aus C₁₋₆-Alkyl oder H sind, sowie Salzen, Estern oder Derivaten davon, vorzugsweise Benzoesäure, Phenylmalonsäure, Benzylmalonsäure, Phenylbernsteinsäure, Benzylbernsteinsäure, Methyl-3-Benzoylpropionat, (S)-3-Phenylbuttersäure und Benzylcarbamat, und Kombinationen davon bestehenden Gruppe ausgewählt ist, kombiniert werden, um die Stabilität der Protease in Wasch- und Reinigungsmitteln weiter zu erhöhen.

Unter der Bezeichnung "Phenylboronsäurederivat" wird eine Verbindung mit der Formel (III) verstanden. 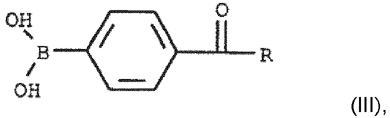 wobei R Wasserstoff, eine Hydroxyl-, eine C₁₋₆ Alkyl-, eine substituierte C₁₋₆ Alkyl-, eine C₁₋₆ Alkenyl oder eine substituierte C₁₋₆ Alkenyl-Gruppe ist. Bevorzugt ist der Rest R in dem Phenylboronsäurederivat eine C₁₋₆ Alkyl-Gruppe und hierunter weiter bevorzugt -CH₃, -CH₃CH₂ oder - CH₃CH₂CH₂ sein. Weiter bevorzugt ist der Rest R in dem Phenylboronsäurederivat Wasserstoff. Besonders bevorzugt ist das Phenylboronsäurederivat 4-Formyl-phenylboronsäure (4-FPBA).

Die eingesetzte reversible Inhibitorverbindung kann Borsäure sein.

Die eingesetzte reversible Inhibitorverbindung kann eine Verbindung der Formeln (I) oder (II) sein. 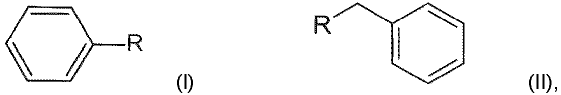 wobei R jeweils ausgewählt ist aus -COOH, C₁₋₆-Alkyl-substituierten oder unsubstituierten C₂₋₆-Dicarbonsäuren, C₁₋₆-Alkyl-substituierten oder unsubstituierten C₂₋₆-Carbonsäuren und -OOC-NR²₂, wobei R² gleich oder verschieden und ausgewählt aus C₁₋₆-Alkyl oder H sind, sowie Salzen, Estern oder Derivaten davon, und Kombinationen davon bestehenden Gruppe ausgewählt ist, sein, vorzugsweise ausgewählt aus der aus Benzoesäure, Phenylmalonsäure, Benzylmalonsäure, Phenylbernsteinsäure, Benzylbernsteinsäure, Methyl-3-Benzoylpropionat, (S)-3-Phenylbuttersäure und Benzylcarbamat bestehenden Gruppe.

Die zweite Waschmittelzusammensetzung ist vorzugsweise im Wesentlichen frei von borhaltigen Verbindungen. "Im Wesentlichen frei von borhaltigen Verbindungen" bedeutet in diesem Zusammenhang, dass die zweite Waschmittelzusammensetzung, bezogen auf ihr Gesamtgewicht, weniger als 2 Gew.-%, vorzugsweise weniger als 1 Gew.-%, weiter bevorzugt weniger als 0,5 Gew.-% und besonders bevorzugt weniger als 0,1 Gew.-%, borhaltige Verbindungen, enthält. In einer ganz besonders bevorzugten Ausführungsformen ist die zweite Waschmittelzusammensetzung frei von borhaltigen Verbindungen, d.h. sie enthalten keine borhaltigen Verbindungen, insbesondere keine Borsäure und/oder Phenylboronsäurederivate.

Die zweite Waschmittelzusammensetzung wird in Schritt e) des Verfahrens vorzugsweise aus einem in die Waschmaschine integrierten Behälter dosiert wird, welcher mit der mehrfachen des für die Durchführung eines Waschprogramms notwendigen Menge der zweiten Waschmittelzusammensetzung befüllt ist.

Die Dosierung der zweiten Waschmittelzusammensetzung in Schritt e) erfolgt vorzugsweise derart, dass die zweite Waschmittelzusammensetzung innerhalb eines Zeitraums von fünf Minuten, besonders bevorzugt von 2 Minuten und insbesondere von 1 Minute in die wässrige Flotte eingebracht wird.

Die wässrige Flotte weist in Schritt e) vorzugsweise eine Temperatur von 26 bis 64°C auf.

Nach Beendigung des Hauptwaschgangs zum Zeitpunkt 100% t_{w} wird die wässrige Flotte vorzugsweise aus dem Wäschebehandlungsraum abgepumpt.

Die Zusammensetzung einiger bevorzugter erster und zweiter Waschmittelzusammensetzungen für den Einsatz in dem Verfahren kann den folgenden Tabellen entnommen werden, wobei die Zusammensetzungen nicht zwingend gemäss der Erfindung sind. (Angaben in Gew.-% bezogen auf das Gesamtgewicht des Gelkörpers bzw. der Hüllsubstanz sofern nicht anders angegeben).

| | Formel 1 | Formel 2 | Formel 3 | Formel 4 |
|---|---|---|---|---|
| **Erste Waschmittelzusammensetzung** | | | | |
| Tensid | 50 bis 95 | 55 bis 92 | 55 bis 92 | 60 bis 88 |
| Misc | ad 100 | ad 100 | ad 100 | ad 100 |

| **Zweite Waschmittelzusammensetzung** | | | | |
|---|---|---|---|---|
| aktives Protease-Protein | 4 bis 18 | 4 bis 18 | 5 bis 15 | 7 bis 12 |
| flüssiger Träger | 80 bis 96 | 80 bis 96 | 85 bis 95 | 85 bis 90 |

| | Formel 5 | Formel 6 | Formel 7 | Formel 8 |
|---|---|---|---|---|
| **Erste Waschmittelzusammensetzung** | | | | |
| Tensid | 50 bis 95 | 55 bis 92 | 55 bis 92 | 60 bis 88 |
| Misc | ad 100 | ad 100 | ad 100 | ad 100 |

| **Zweite Waschmittelzusammensetzung** | | | | |
|---|---|---|---|---|
| aktives Protease-Protein* | 4 bis 18 | 4 bis 18 | 5 bis 15 | 7 bis 12 |
| flüssiger Träger | 80 bis 96 | 80 bis 96 | 85 bis 95 | 85 bis 90 |

| | Formel 11 | Formel 12 | Formel 13 | Formel 14 |
|---|---|---|---|---|
| **Erste Waschmittelzusammensetzung** | | | | |
| Aniontensid | 30 bis 90 | 30 bis 90 | 40 bis 80 | 45 bis 70 |
| Niotensid | 7 bis 50 | 12 bis 40 | 12 bis 40 | 15 bis 30 |
| Misc | ad 100 | ad 100 | ad 100 | ad 100 |

| **Zweite Waschmittelzusammensetzung** | | | | |
|---|---|---|---|---|
| aktives Protease-Protein | 4 bis 18 | 4 bis 18 | 5 bis 15 | 7 bis 12 |
| flüssiger Träger | 80 bis 96 | 80 bis 96 | 85 bis 95 | 85 bis 90 |

| | Formel 15 | Formel 16 | Formel 17 | Formel 18 |
|---|---|---|---|---|
| **Erste Waschmittelzusammensetzung** | | | | |
| Aniontensid | 12 bis 40 | 15 bis 30 | 15 bis 30 | 18 bis 25 |
| Niotensid | 12 bis 40 | 15 bis 30 | 15 bis 30 | 18 bis 25 |
| Misc | ad 100 | ad 100 | ad 100 | ad 100 |

| **Zweite Waschmittelzusammensetzung** | | | | |
|---|---|---|---|---|
| aktives Protease-Protein* | 4 bis 18 | 4 bis 18 | 5 bis 15 | 7 bis 12 |
| flüssiger Träger | 80 bis 96 | 80 bis 96 | 85 bis 95 | 85 bis 90 |

| | | | | |
|---|---|---|---|---|
| * wobei das aktive Protease-Protein proteolytische Aktivität aufweist und eine Aminosäuresequenz umfasst, die mindestens 70% Sequenzidentität mit der in SEQ ID NO:1 angegebenen Aminosäuresequenz über deren Gesamtlänge aufweist und jeweils bezogen auf die Nummerierung gemäß SEQ ID NO:1 (i) an mindestens zwei, vorzugsweise drei der Positionen, die den Positionen 3, 4, 99 oder 199 entsprechen, mindestens zwei Aminosäuresubstitutionen, vorzugsweise drei Aminosäuresubstitutionen, ausgewählt aus den Aminosäuresubstitutionen 3T, 4I, 99E und 199I, und (ii) an mindestens einer der Positionen, die den Positionen 74, 136, 143, 154, 160, 161, 163, 171, 181, 183, 185, 200, 203, 209, 212 oder 256 entsprechen, mindestens eine weitere Aminosäuresubstitution, insbesondere ausgewählt aus N74D/E/Q, A136Q, R143L/W/Y, S154D/Q, S160G, Y161T, A163G, V171L, A181D, F183R, Q185R, Q200A/L/S/T, Y203K/V/W, A209K/W, N212S/T und L256D/E/Q, aufweist | | | | |

## Patentansprüche

1. Waschverfahren für Textilien in einer Trommelwaschmaschine, umfassend die Schritte
a) Bereitstellen einer Waschmaschine mit einem Waschprogramm, umfassend einen Hauptwaschgang der Dauer t_{w};
b) Einbringen von Textilien in den Wäschebehandlungsraum der Waschmaschine;
c) Einbringen einer wässrigen Flotte in den Wäschebehandlungsraum der Waschmaschine;
d) Einbringen einer ersten Waschmittelzusammensetzung in den Wäschebehandlungsraum der Waschmaschine im Hauptwaschgang zu einem Zeitpunkt 0 bis 10% t_{w};
e) Einbringen einer zweiten Waschmittelzusammensetzung, umfassend, bezogen auf ihr Gesamtgewicht,
i) mindestens 4 Gew.-% aktives Protease-Protein
ii) mindestens 80 Gew.-% eines flüssigen Trägers
in die wässrige Flotte des Hauptwaschgangs zu einem Zeitpunkt 11 bis 99% t_{w},
wobei die erste Waschmittelzusammensetzung, bezogen auf ihr Gesamtgewicht
i) 40 bis 80 Gew.-% anionisches Tensid aus der Gruppe der C₈₋₁₈-Alkylbenzolsulfonsäuren, der Alkylethersulfonsäuren und der Fettsäuren,
ii) 12 bis 40 Gew.-% nichtionisches Tensid aus der Gruppe der ethoxylierten primären C6-18-Alkohole,
iii) 4,5 bis 27 Gew.-% organisches Lösungsmittel aus der Gruppe der organischen Amine,
iv) weniger als 6 Gew.-% und insbesondere weniger als 2 Gew.-% Wasser enthält.

2. Waschverfahren nach Anspruch 1, wobei die Dauer t_{w} des Hauptwaschgangs 15 bis 400 Minuten, vorzugsweise 30 bis 240 Minuten und insbesondere 60 bis 180 Minuten beträgt.

3. Waschverfahren nach einem der vorherigen Ansprüche, wobei die wässrige Flotte in Schritt c) eine Wasserhärte oberhalb 14°dH, vorzugsweise oberhalb 17°dH und insbesondere oberhalb 21°dH aufweist.

4. Waschverfahren nach einem der vorherigen Ansprüche, wobei die Trommelwaschmaschine über eine Vorrichtung zur Erfassung der Wasserhärte der in Schritt c) eingesetzten wässrigen Flotte verfügt, wobei das Waschprogramm vorzugsweise derart ausgestaltet ist, dass die mittels der Vorrichtung erfassten Wasserhärten vorzugsweise die Menge der in Schritt e) eingebrachten zweiten Waschmittelzusammensetzung beeinflusst.

5. Waschverfahren nach einem der vorherigen Ansprüche, wobei die erste Waschmittelzusammensetzung, bezogen auf ihr Gesamtgewicht
i) 45 bis 70 Gew.-% anionisches Tensid aus der Gruppe der C₈₋₁₈-Alkylbenzolsulfonsäuren, der Alkylethersulfonsäuren und der Fettsäuren,
ii) 15 bis 30 Gew.-% nichtionisches Tensid aus der Gruppe der ethoxylierten primären C6-18-Alkohole,
iii) 10 bis 24 Gew.-% organisches Lösungsmittel aus der Gruppe der organischen Amine
iv) weniger als 2 Gew.-% Wasser enthält.

6. Waschverfahren nach einem der vorherigen Ansprüche, wobei die erste Waschmittelzusammensetzung in Schritt d) aus einem in die Waschmaschine integrierten Behälter dosiert wird, welcher mit der mehrfachen des für die Durchführung eines Waschprogramms notwendigen Menge der ersten Waschmittelzusammensetzung befüllt ist.

7. Waschverfahren nach einem der vorherigen Ansprüche, wobei die zweite Waschmittelzusammensetzung, bezogen auf ihr Gesamtgewicht, 4 bis 18 Gew.-%, vorzugsweise 5 bis 15 Gew.-% und insbesondere 7 bis 12 Gew.-% aktives Protease-Protein umfasst.

8. Waschverfahren nach einem der vorherigen Ansprüche, wobei das aktive Protease-Protein proteolytische Aktivität aufweist und eine Aminosäuresequenz umfasst, die mindestens 70% Sequenzidentität mit der in SEQ ID NO:1 angegebenen Aminosäuresequenz über deren Gesamtlänge aufweist und jeweils bezogen auf die Nummerierung gemäß SEQ ID NO:1 (i) an mindestens zwei, vorzugsweise drei der Positionen, die den Positionen 3, 4, 99 oder 199 entsprechen, mindestens zwei Aminosäuresubstitutionen, vorzugsweise drei Aminosäuresubstitutionen, ausgewählt aus den Aminosäuresubstitutionen 3T, 4I, 99E und 199I, und (ii) an mindestens einer der Positionen, die den Positionen 74, 136, 143, 154, 160, 161, 163, 171, 181, 183, 185, 200, 203, 209, 212 oder 256 entsprechen, mindestens eine weitere Aminosäuresubstitution, insbesondere ausgewählt aus N74D/E/Q, A136Q, R143L/W/Y, S154D/Q, S160G, Y161T, A163G, V171L, A181D, F183R, Q185R, Q200A/L/S/T, Y203K/V/W, A209K/W, N212S/T und L256D/E/Q, aufweist, vorzugsweise in einer Menge von 0,0001 bis 1 Gew.-%, bezogen auf das Gesamtgewicht des Wasch- und Reinigungsmittels,

9. Waschverfahren nach einem der vorherigen Ansprüche, wobei die zweite Waschmittelzusammensetzung, bezogen auf ihr Gesamtgewicht, 80 bis 96 Gew.-%, vorzugsweise 85 bis 95 Gew.-% und insbesondere 85 bis 90 Gew.-% flüssigen Träger umfasst.

10. Waschverfahren nach einem der vorherigen Ansprüche, wobei die zweite Waschmittelzusammensetzung als flüssigen Träger ein wässrig-organisches Lösungsmittelgemisch aufweist.

11. Waschverfahren nach einem der vorherigen Ansprüche, wobei der flüssige Träger der zweiten Waschmittelzusammensetzung organisches Lösungsmittel aus der Gruppe Ethanol, n-Propanol, i-Propanol, Butanolen, Glykol, Propandiol, Butandiol, Methylpropandiol, Glycerin, Diglykol, Propyldiglycol, Butyldiglykol, Hexylenglycol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropy-lenglykolmonoethylether, Methoxytriglykol, Ethoxytriglykol, Butoxytriglykol, 1-Butoxyethoxy-2-propanol, 3-Methyl-3-methoxybutanol, Propylen-glykol-t-butylether, Di-n-octylether sowie deren Mischungen, vorzugsweise aus der Gruppe Propandiol, Glycerin und Ethanol umfasst.

12. Waschverfahren nach einem der vorherigen Ansprüche, wobei die zweite Waschmittelzusammensetzung im Wesentlichen frei von borhaltigen Verbindungen, vorzugsweise frei von borhaltigen Verbindungen ist

13. Waschverfahren nach einem der vorherigen Ansprüche, wobei die zweite Waschmittelzusammensetzung in Schritt e) aus einem in die Waschmaschine integrierten Behälter dosiert wird, welcher mit der mehrfachen des für die Durchführung eines Waschprogramms notwendigen Menge der zweiten Waschmittelzusammensetzung befüllt ist.

14. Waschverfahren nach einem der vorherigen Ansprüche, wobei die zweite Waschmittelzusammensetzung in Schritt e) zu einem Zeitpunkt 20 bis 70% t_{w}, vorzugsweise zu einem Zeitpunkt 25 bis 65% t_{w}, besonders bevorzugt zu einem Zeitpunkt 30 bis 60% t_{w} in die wässrige Flotte eingebracht wird.

## Claims

1. Method for washing textiles in a drum washing machine, comprising the steps of
a) providing a washing machine with a washing program comprising a main wash cycle of duration t_{w} ;
b) introducing textiles into the laundry treatment chamber of the washing machine;
c) introducing an aqueous liquor into the laundry treatment chamber of the washing machine;
d) int a first detergent composition into the laundry treatment chamber of the washing machine during the main wash cycle at a time 0 to 10% t_{w} ;
e) Introducing a second detergent composition comprising, based on its total weight,
i) at least 4% by weight of active protease protein
ii) at least 80% by weight of a liquid carrier
into the aqueous liquor of the main wash cycle at a time 11 to 99% t_{w} ,
wherein the first detergent composition comprises, based on its total weight
i) 40 to 80% by weight of anionic surfactant from the group of C₈₋₁₈alkylbenzenesulfonic acids, alkyl ether sulfonic acids and fatty acids,
ii) 12 to 40% by weight of non-ionic surfactant from the group of ethoxylated primary C6-18 alcohols,
iii) 4.5 to 27 wt% organic solvent from the group of organic amines,
iv) less than 6 wt.% and, in particular, less than 2 wt.% water.

2. Washing method according to claim 1, wherein the duration t_{w}of the main washing cycle is 15 to 400 minutes, preferably 30 to 240 minutes and in particular 60 to 180 minutes.

3. Washing method according to one of the preceding claims, wherein the aqueous liquor in step c) has a water hardness above 14°dH, preferably above 17°dH and in particular above 21°dH.

4. Washing method according to any one of the preceding claims, wherein the drum washing machine is provided with a device for detecting the water hardness of the aqueous liquor used in step c), and wherein the washing program is preferably configured such that the water hardness values detected by the device preferably influence the amount of the second detergent composition introduced in step e).

5. Washing method according to one of the preceding claims, wherein the first detergent composition, based on its total weight
i) 45 to 70% by weight of anionic surfactant from the group of C₈₋₁₈alkylbenzenesulfonic acids, alkyl ether sulfonic acids and fatty acids,
ii) 15 to 30 wt% non-ionic surfactant from the group of ethoxylated primary C6-18 alcohols,
iii) 10 to 24 wt% organic solvent from the group of organic amines
iv) less than 2% by weight of water.

6. Washing method according to one of the preceding claims, wherein the first detergent composition in step d) is dispensed from a container integrated into the washing machine, which is filled with a multiple of the amount of the first detergent composition required to carry out a washing programme.

7. Washing method according to one of the preceding claims, wherein the second detergent composition comprises 4 to 18 wt.%, preferably 5 to 15 wt.% and in particular 7 to 12 wt.% active protease protein, based on its total weight.

8. Washing method according to one of the preceding claims, wherein the active protease protein has proteolytic activity and comprises an amino acid sequence which has at least 70% sequence identity with the amino acid sequence specified in SEQ ID NO:1 over its entire length and, in each case relative to the numbering according to SEQ ID NO:1 (i) at least two, preferably three, of the positions corresponding to positions 3, 4, 99 or 199, at least two amino acid substitutions, preferably three amino acid substitutions, selected from the amino acid substitutions 3T, 4I, 99E and 199I, and (ii) at least one further amino acid substitution at at least one of the positions corresponding to positions 74, 136, 143, 154, 160, 161, 163, 171, 181, 183, 185, 200, 203, 209, 212 or 256, at least one further amino acid substitution, in particular selected from N74D/E/Q, A136Q, R143L/W/Y, S154D/Q, S160G, Y161T, A163G, V171L, A181D, F183R, Q185R, Q200A/L/S/T, Y203K/V/W, A209K/W, N212S/T and L256D/E/Q, preferably in an amount of 0.0001 to 1% by weight, based on the total weight of the detergent and cleaning agent,

9. Washing method according to one of the preceding claims, wherein the second detergent composition comprises 80 to 96 wt.%, preferably 85 to 95 wt.% and in particular 85 to 90 wt.% liquid carrier, based on its total weight.

10. Washing method according to one of the previous claims, wherein the second detergent composition comprises an aqueous-organic solvent mixture as liquid carrier.

11. Washing method according to one of the preceding claims, wherein the liquid carrier of the second detergent composition comprises an organic solvent from the group consisting of ethanol, n-propanol, i-propanol, butanols, glycol, propanediol, butanediol, methylpropanediol, glycerine, diglycol, propyldiglycol, butyldiglycol, hexyleneglycol, ethyleneglycol methyl ether, ethyleneglycol ethyl ether, ethyleneglycol propyl ether, ethyleneglycol mono-n-butyl ether, diethyleneglycol methyl ether, diethylene glycol ethyl ether, propylene glycol methyl ether, propylene glycol ethyl ether, propylene glycol propyl ether, dipropylene glycol monomethyl ether, dipropylene glycol monoethyl ether, methoxy triglycol, ethoxy triglycol, butoxytriglycol, 1-butoxyethoxy-2-propanol, 3-methyl-3-methoxybutanol, propylene glycol t-butyl ether, di-n-octyl ether and mixtures thereof, preferably from the group comprising propanediol, glycerine and ethanol.

12. Washing method according to one of the preceding claims, wherein the second detergent composition is essentially free of boron-containing compounds, preferably free of boron-containing compounds

13. Washing method according to one of the preceding claims, wherein the second detergent composition is dosed in step e) from a container integrated into the washing machine, which is filled with a multiple of the amount of the second detergent composition necessary for carrying out a washing programme.

14. Washing method according to one of the preceding claims, wherein the second detergent composition is introduced into the aqueous liquor in step e) at a time 20 to 70% t_{w}, preferably at a time 25 to 65% t_{w}, particularly preferably at a time 30 to 60% t_{w}.

## Revendications

1. Procédé de lavage de textiles dans une machine à laver à tambour, comprenant les étapes
a) fournir une machine à laver avec un programme de lavage comprenant un cycle de lavage principal d'une durée t_{w} ;
b) introduction de textiles dans la chambre de traitement du linge de la machine à laver ;
c) introduction d'un bain aqueux dans la chambre de traitement du linge de la machine à laver ;
d) l' d'une première composition détergente dans la chambre de traitement du linge de la machine à laver pendant le cycle de lavage principal à un moment compris entre 0 et 10 % t_{w} ;
e) introduction d'une deuxième composition détergente comprenant, par rapport à son poids total,
i) au moins 4 % en poids de protéase active
ii) au moins 80 % en poids d'un support liquide
dans le bain aqueux du cycle de lavage principal à un moment compris entre 11 et 99 % t_{w},
la première composition détergente comprenant, par rapport à son poids total
i) 40 à 80 % en poids de tensioactif anionique choisi dans le groupe des acides alkylbenzènesulfoniques en C₈₋₁₈, des acides alkyléthersulfoniques et des acides gras,
ii) 12 à 40 % en poids de tensioactif non ionique choisi dans le groupe des alcools primaires en C6-18 éthoxylés,
iii) 4,5 à 27 % en poids de solvant organique choisi dans le groupe des amines organiques,
iv) moins de 6 % en poids et en particulier moins de 2 % en poids d'eau.

2. Procédé de lavage selon la revendication 1, dans lequel la durée t_{w} du cycle de lavage principal est comprise entre 15 et 400 minutes, de préférence entre 30 et 240 minutes et en particulier entre 60 et 180 minutes.

3. Procédé de lavage selon l'une des revendications précédentes, dans lequel le liquide aqueux de l'étape c) présente une dureté de l'eau supérieure à 14 °dH, de préférence supérieure à 17 °dH et en particulier supérieure à 21 °dH.

4. Procédé de lavage selon l'une des revendications précédentes, la machine à laver à tambour disposant d'un dispositif de détection de la dureté de l'eau de la flotte aqueuse utilisée à l'étape c), le programme de lavage étant de préférence conçu de manière que les duretés de l'eau détectées au moyen du dispositif influencent de préférence la quantité de la seconde composition détergente introduite à l'étape e).

5. Procédé de lavage selon l'une des revendications précédentes, dans lequel la première composition détergente, par rapport à son poids total
i) 45 à 70 % en poids de tensioactif anionique choisi dans le groupe des acides C₈₋₁₈-alkylbenzènesulfoniques, des acides alkyléthersulfoniques et des acides gras,
ii) 15 à 30 % en poids de tensioactif non ionique choisi dans le groupe des alcools primaires en C6-18 éthoxylés,
iii) 10 à 24 % en poids de solvant organique choisi dans le groupe des amines organiques
iv) moins de 2 % en poids d'eau.

6. Procédé de lavage selon l'une des revendications précédentes, dans lequel la première composition détergente est dosée à l'étape d) à partir d'un récipient intégré dans la machine à laver, qui est rempli d'une quantité de la première composition détergente plusieurs fois supérieure à la quantité nécessaire pour exécuter un programme de lavage.

7. Procédé de lavage selon l'une des revendications précédentes, dans lequel la deuxième composition détergente comprend, par rapport à son poids total, 4 à 18 % en poids, de préférence 5 à 15 % en poids et en particulier 7 à 12 % en poids de protéase active.

8. Procédé de lavage selon l'une des revendications précédentes, dans lequel la protéase active présente une activité protéolytique et comprend une séquence d'acides aminés qui présente au moins 70 % d'identité de séquence avec la séquence d'acides aminés indiquée dans SEQ ID NO:1 sur toute sa longueur et qui, par rapport à la numérotation selon SEQ ID NO:1 (i) au moins deux, de préférence trois substitutions d'acides aminés, de préférence trois substitutions d'acides aminés, choisies parmi les substitutions d'acides aminés 3T, 4I, 99E et 199I, et (ii) à au moins une des positions correspondant aux positions 74, 136, 143, 154, 160, 161, 163, 171, 181, 183, 185, 200, 203, 209, 212 ou 256, au moins une autre substitution d'acide aminé, en particulier choisie parmi N74D/E/Q, A136Q, R143L/W/Y, S154D/Q, S160G, Y161T, A163G, V171L, A181D, F183R, Q185R, Q200A/L/S/T, Y203K/V/W, A209K/W, N212S/T et L256D/E/Q, de préférence en une quantité de 0,0001 à 1 % en poids par rapport au poids total du détergent et du produit de nettoyage,

9. Procédé de lavage selon l'une des revendications précédentes, dans lequel la deuxième composition détergente comprend, par rapport à son poids total, 80 à 96 % en poids, de préférence 85 à 95 % en poids et en particulier 85 à 90 % en poids, de support liquide.

10. Procédé de lavage selon l'une des revendications précédentes, dans lequel la deuxième composition détergente comprend, en tant que support liquide, un mélange aqueux de solvants organiques.

11. Procédé de lavage selon l'une des revendications précédentes, dans lequel le support liquide de la deuxième composition détergente comprend un solvant organique choisi dans le groupe constitué par l'éthanol, le n-propanol, l'i-propanol, les butanols, le glycol, le propanediol, le butanediol, le méthylpropanediol, glycérine, diglycol, propyldiglycol, butyldiglycol, hexylèneglycol, éther méthylique d'éthylèneglycol, éther éthylique d'éthylèneglycol, éther propylique d'éthylèneglycol, éther mono-n-butylique d'éthylèneglycol, éther méthylique de diéthylèneglycol, diéthylène glycol éthyl éther, propylène glycol méthyl éther, propylène glycol éthyl éther, propylène glycol propyl éther, dipropylène glycol monométhyl éther, dipropylène glycol monoéthyl éther, méthoxy triglycol, éthoxy triglycol, butoxytriglycol, 1-butoxyéthoxy-2-propanol, 3-méthyl-3-méthoxybutanol, propylène glycol t-butyléther, di-n-octyléther ainsi que leurs mélanges, de préférence issus du groupe propanediol, glycérine et éthanol.

12. Procédé de lavage selon l'une des revendications précédentes, dans lequel la deuxième composition détergente est essentiellement exempte de composés contenant du bore, de préférence exempte de composés contenant du bore

13. Procédé de lavage selon l'une des revendications précédentes, dans lequel la deuxième composition détergente est dosée à l'étape e) à partir d'un récipient intégré dans la machine à laver, qui est rempli d'une quantité de la deuxième composition détergente plusieurs fois supérieure à la quantité nécessaire pour exécuter un programme de lavage.

14. Procédé de lavage selon l'une des revendications précédentes, dans lequel la deuxième composition détergente est introduite dans le bain aqueux à un moment compris entre 20 et 70 % t_{w} , de préférence à un moment compris entre 25 et 65 % t_{w} , et de manière particulièrement préférée à un moment compris entre 30 et 60 % t_{(w) ,}lors de l'étape e).
